Europäisches Patentamt

European Patent Office

Office européen des brevets

(11) Veröffentlichungsnummer: **0 312 942**
**A2**

## EUROPÄISCHE PATENTANMELDUNG

(21) Anmeldenummer: 88117188.8

(22) Anmeldetag: 15.10.88

(51) Int. Cl.⁴: **C12N 9/72 , C12N 15/00 , A61K 37/02**

---

Die Bezeichnung der Erfindung wurde geändert (Richtlinien für die Prüfung im EPA, A-III, 7.3).

(30) Priorität: 23.10.87 DE 3735916

(43) Veröffentlichungstag der Anmeldung:
26.04.89 Patentblatt 89/17

(84) Benannte Vertragsstaaten:
AT BE CH DE ES FR GB IT LI NL

(71) Anmelder: BASF Aktiengesellschaft
Carl-Bosch-Strasse 38
D-6700 Ludwigshafen(DE)

(72) Erfinder: Koerwer, Wolfgang, Dr.
Auf dem Leimen 10
D-6718 Grünstadt(DE)
Erfinder: Kurfürst, Manfred, Dr.
Anilinstrasse 71
D-6733 Hassloch(DE)
Erfinder: Baldinger, Verena, Dr.
Schiffsgasse 6
D-6900 Heidelberg(DE)
Erfinder: Doerper, Thomas, Dr.
Luitpoldstrasse 3
D-6719 Bissersheim(DE)

---

(54) **Proteine mit Prourokinase-Aktivität, ihre Herstellung und Verwendung.**

(57) Es werden Polypeptide beschrieben, die sich von Prourokinase durch den Austausch einer Aminosäure unterscheiden. Die Polypeptide werden gentechnisch hergestellt und besitzen thrombolytische Eigenschaften.

EP 0 312 942 A2

EP 0 312 942 A2

## Neue Polypeptide, ihre Herstellung und Verwendung

Die vorliegende Erfindung betrifft neue Polypeptide mit Prourokinase-Aktivität, Verfahren zu deren Herstellung und deren Verwendung bei der Bekämpfung von Krankheiten.

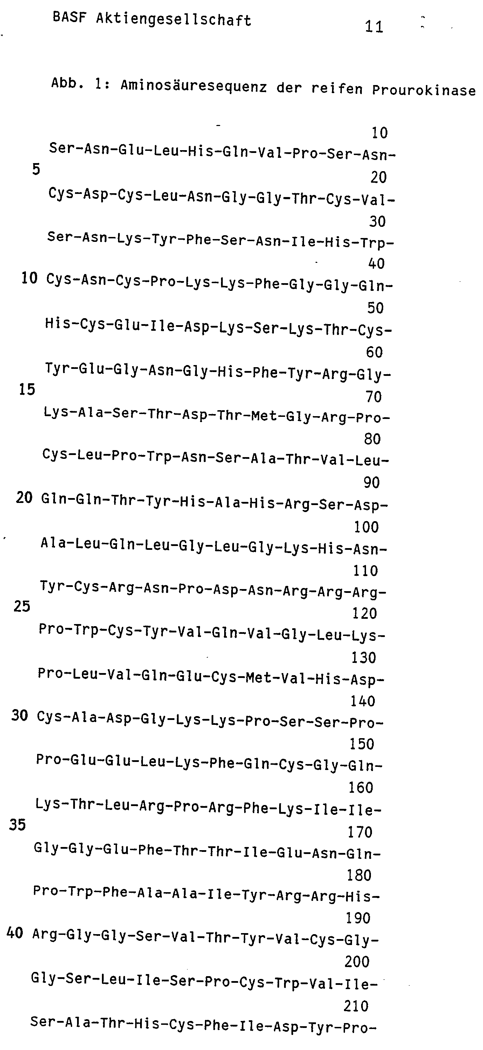

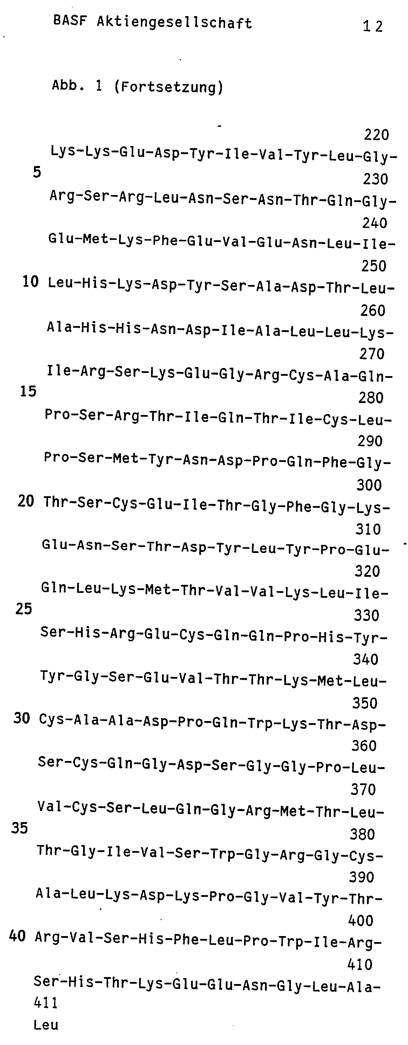

Der Plasminogenaktivator Prourokinase (PUK), auch "single chain urokinase plasminogenactivator (SCUPA)" oder "kidney plasminogenactivator" (KPA) genannt, ist ein Einketten-Polypeptid aus 411 Aminosäuren und einem Molekulargewicht von etwa 54 kD. Abb. 1 zeigt die Aminosäuresequenz der reifen Prourokinase (Z. Physiol. Chem. 363, 133, 1043, 1155 (1982)). Aus der Prourokinse entsteht durch limitierte Proteasespaltung der $Lys^{157}$-$Phe\overline{158}$-Bindung ein Zweikettenmolekül, die hochmolekulare Urokinase (HUK). Diese besitzt dasselbe Molekulargewicht wie die Prourokinase. Durch eine weitere proteolytische Spaltung zwischen $Lys^{135}$ und $Lys^{136}$ entsteht die niedermolekulare Urokinase (LUK) mit einem Molekulargewicht von etwa 33 kD. Die LUK ist wie die HUK ein Zweikettenmolekül. Alle drei Formen sind im humanen Urin oder im humanen Serum als natürliche Formen nachgewiesen worden (J. Biol. Chem. 257, 3276 (1982) und 261, 1267 (1986)).

Sowohl die Ein-, als auch die Zweikettenformen besitzen fibrinolytische Eigenschaften. Die Zweikettenmoleküle wirken allerdings systemisch. Sie degradieren sowohl Fibrin als auch Fibrinogen überall im Körper, und es kommt bei der Applikation häufig zu lebensbedrohenden Blutungen. Allein die Einkettenformen sind gerinnselspezifisch und zeigen nur eine geringe systemische Lyse.

Es wurde nun gefunden, daß Polypeptide der Formel I

$$R\text{-}PUK^{1\text{-}155}\text{-}X\text{-}PUK^{157\text{-}411}$$

worin R ein Wasserstoffatom, Ala oder Met,
$PUK^{1\text{-}155}$ und $PUK^{157\text{-}411}$ für die Aminosäuren 1 bis 155 und 157 bis 411 der Prourokinase und
X Ala, Asn, Asp, Cys, Gln, Glu, Gly, His, Ile, Leu, Lys, Met, Phe, Pro, Ser, Thr, Trp, Try oder Val darstellen, verbesserte Eigenschaften besitzen und enzymatisch stabiler sind.

Die Polypeptide der Formel I können an der Aminosäure 302 (Asn, vgl. Abb. 1) einen Glykosylrest tragen.

Die Aminosäurereste 1 bis 155 und 157 bis 411 der Prourokinase sind aus Abb. 1 ersichtlich.

Die Erfindung betrifft weiter DNA-Sequenzen, die für die Polypeptide der Formel I kodieren, sowie Vektoren, die diese DNA-Sequenzen enthalten.

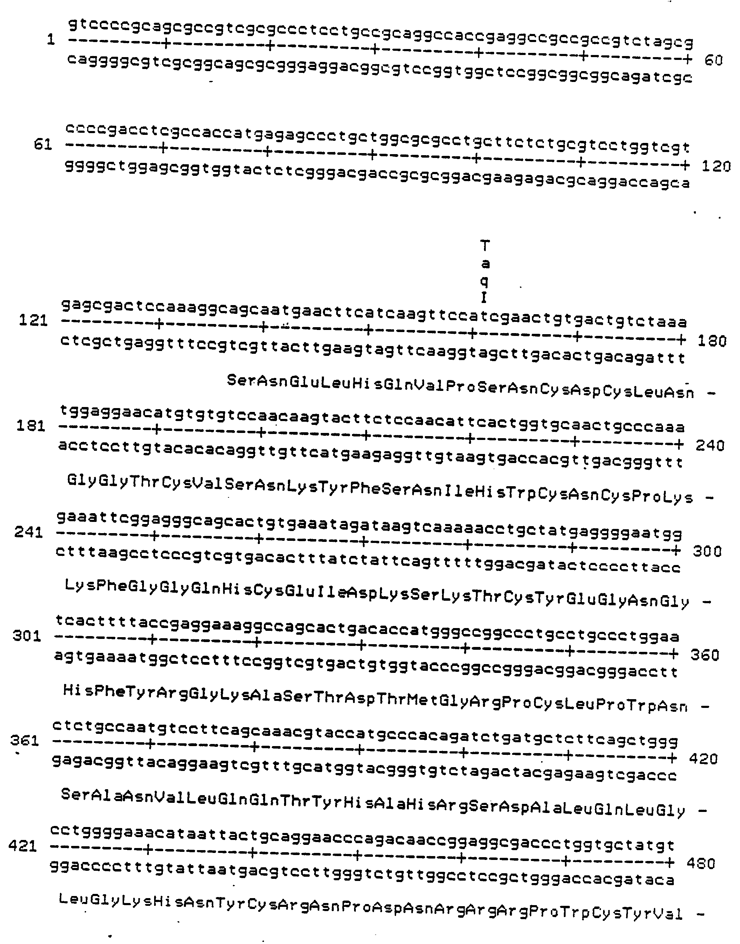

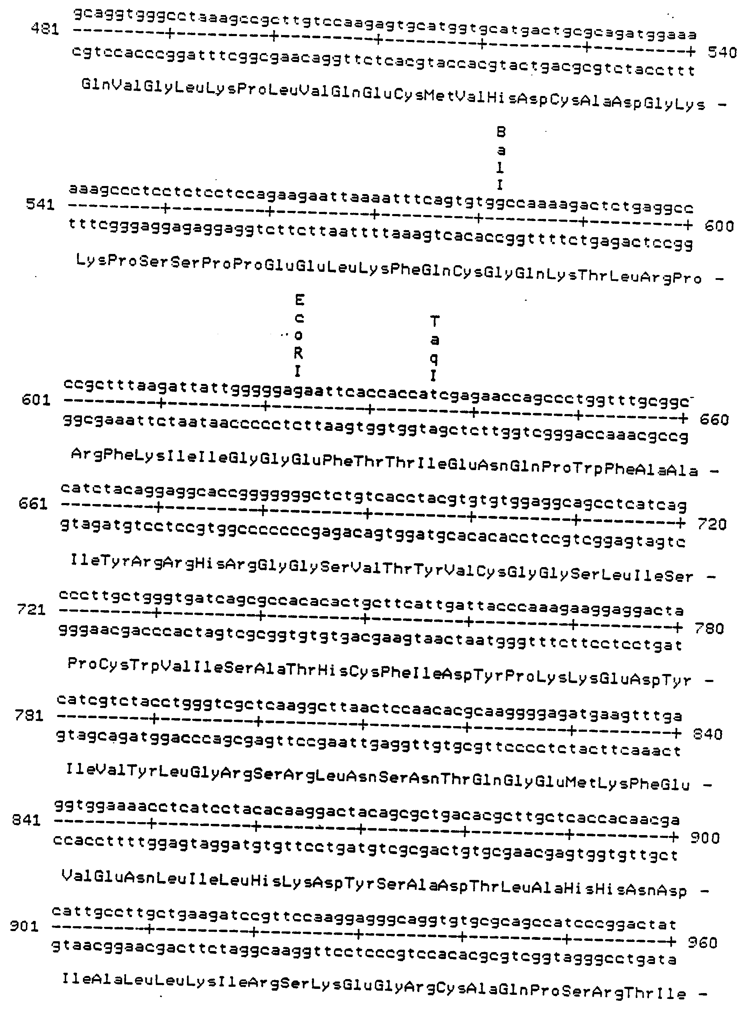

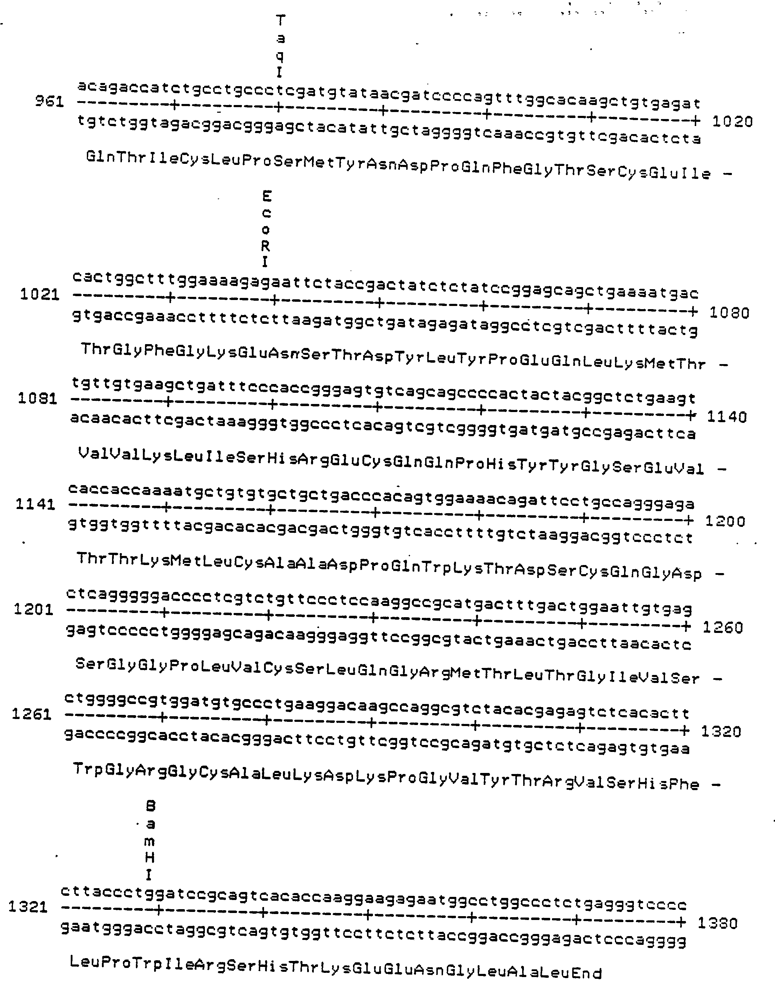

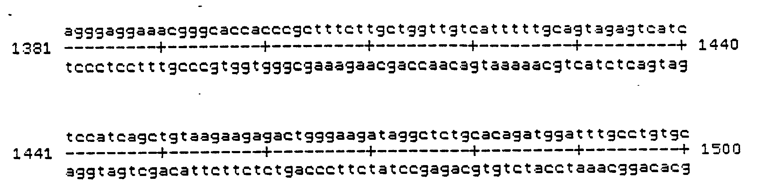

Die neuen Polypeptide lassen sich gentechnologisch nach bekannten Verfahren herstellen. Ausgangspunkt für die hier beschriebenen Konstruktionen ist ein cDNA-Klon, im folgenden pU20 genannt, der die kodierende Sequenz von Prourokinase und noch 5′- und 3′- nicht translatierte Bereiche beinhaltet. Die Teilsequenz des cDNA-Klons, der die kodierende Region umfaßt und aus der sich die Proteinsequenz (Abb. 1) ableitet, ist in Abb. 2 dargestellt. Das reife Protein beginnt nach einer sogenannten "leader"-Sequenz bei $Ser^1$ und terminiert nach $Leu^{411}$.

pU20 wurde aus der humanen, Prourokinase produzierenden Tumorzellinie Detroit 562 (ATCC No. CCL 138) erhalten: Aus dieser Zellinie wurde m-RNA isoliert und in doppelsträngige cDNA umgeschrieben.

Nach Einsetzen dieser cDNA in den kommerziell erhältlichen Klonierungsvektor puc 18 wurde eine cDNA-Bibliothek angelegt. Die dabei verwendeten Methoden sind beispielsweise in Maniatis et al, "Molecular Cloning", CSH-Press nachzulesen. Auch das "Screenen" solcher Genbänke mit radioaktiv markierten Oligonukleotidsonden ist inzwischen eine vielfach verwendete und beschriebene Methode.

Teile der DNA-Sequenz von pU20 sind mit Hilfe von Restriktionsendonukleasen leicht zugänglich. Diese Fragmente, gegebenenfalls in Verbindung mit chemisch synthetisierten Oligonukleotiden, Adaptoren oder Genfragmenten können benutzt werden, um die DNA-Sequenzen, die für die neuen Polypeptide kodieren, zu klonieren. Der Einbau der Genfragmente bzw. synthetischen DNA-Sequenzen in Klonierungsvektoren, beispielsweise die handelsüblichen Plasmide pBR 322, puc 18 und puc 19, erfolgt in bekannter Weise. Auch können die Gene oder Genfragmente mit geeigneten chemisch synthetisierten oder aus Bakterien bzw. Phagen isolierten Kontrollregionen versehen werden, die die Expression der Proteine ermöglichen. Die Transformation der so erhaltenen Hybridplasmide in geeignete Wirtsorganismen ist ebenfalls bekannt und eingehend beschrieben. Auch können die Hybridplasmide mit entsprechenden Signalsequenzen versehen werden, die die Sekretion der Polypeptide in das Periplasma von E.coli erlauben.

Eine Expression in Mammalia-Zellkultur bzw. Hefe oder Insektenzellen ist in analoger Weise möglich: Hierbei müssen allerdings eukaryontische Kontrollregionen verwendet werden. Bei der Expression in Mammaliazellen kann man Vektoren verwenden, die z.B. das zu exprimierende Fremdgen unter die

2

Kontrolle des viralen SV 40 Promotors setzt. Solche Plasmide besitzen keinen eukaryontischen Replikationsursprung. Man isoliert nach der Transformation in permissive Mammaliazellen Klone, die Kopien dieser Plasmide integriert im Genom tragen. In gleicher Weise kann man die zu exprimierenden Gene unter der Kontrolle eines eukaryontischen Promotors auf Vektoren auf der Basis des Rinderpapillom-Virus klonieren. Solche Expressionsplasmide bleiben episomal in einer Kopienzahl von 20 bis 100 in der permissiven Mausfibroblastenzellinie C127.

Diese eukaryontischen Expressionssysteme besitzen den Vorteil, daß sie in der Lage sind, ihre Produkte effektiv und meist in nativer Form zu sekretieren. Ferner besitzen sie die Fähigkeit, ihre Produkte posttranslationell zu modifizieren.

So erhält die Prourokinase bei einer Expression in Eukaryontenzellen noch eine Glykosidseitenkette an der Aminosäure 302 (Asn). Bakterien sind nicht in der Lage, Glycosidseitenketten zu synthetisieren. Die meisten in Bakterien exprimierten eukaryontischen Proteine, wie auch die Prourokinase und die von ihr erfindungsgemäß abgeleiteten Polypeptide, fallen in der Zelle als denaturierte Einschlußkörper an und müssen proteinchemisch zurückgefaltet werden. Ferner sind Bakterien oft nicht in der Lage, die Initiatoraminosäure Methionin vom fertigen Protein abzuspalten. Die Abspaltung des endständigen Methionins bei der Expression in Bakterien und in manchen Fällen die Verhinderung der Bildung von Einschlußkörpern kann durch die Verwendung von Sekretionssystemen erreicht werden. Bei der Verwendung solcher Systeme besitzt dann allerdings häufig der N-Terminus noch eine für die Spaltstelle wichtige Aminosäure, wie z.B. Alanin. Obwohl die Expression in Bakterien viele Probleme aufwirft, sind diese, wie bei der Prourokinase das Expressionssystem der Wahl, wenn sich das anfallende Rohprotein zum aktiven Enzym renaturieren läßt. Daher wird hier die Expression in Bakterien bevorzugt.

Aufgrund der Degeneration des genetischen Codes ist es auch möglich, andere DNA-Sequenzen, z.B. chemisch synthetisierte Prourokinasegene oder Teile davon mit anderen Tripletts als die hier beschriebenen für die Expression der neuen Polypeptide zu benutzen.

Die neuen Wirkstoffe können zur Thrombolyse bei arteriellen und venösen Verschlußkrankheiten eingesetzt werden und zeigen verbesserte Eigenschaften gegenüber humaner Prourokinase.

Gegenstand der Erfindung sind daher auch Arzneimittel, die mindestens eines der neuen Polypeptide enthalten, gegebenenfalls in einem pharmazeutisch verträglichen Träger oder Bindemittel.

Die neuen Proteine können auch zusammen mit anderen Fibrinolytika, wie Urokinase, TPA, Streptokinase, von diesen drei Parteien abgeleiteten Derivaten oder Natriumpentosanpolysulfat, angewendet werden. Besonders eignen sich TPA und davon abgeleitete Proteine mit ähnlicher Aktivität als Kombinationsbestandteile.

Weitere Ausgestaltungen der Erfindung sind in den folgenden Beispielen näher beschrieben.

Beispiele

## 1. Isolierung eines cDNA-Klons für humane Prourokinase

Die Prourokinase produzierende humane Tumorzellinie Detroit 562 (ATCC Nr. CCL 138) wurde in "Eagle's minimal essential"-Medium mit 3 % $NaHCO_3$, 10 % fötalem Kälberserum, 1 % Aminosäuren, 2,4 % Hepes pH 7,5 bei 37° C und 5 % $CO_2$-Atmosphäre bis zur Konfluenz gezogen. Das Medium wurde abgegossen, die Zellen mit physiologischer Kochsalzlösung gewaschen und in Lysepuffer (6M Guanidiniumisothiocyanat, 5 mM Natriumcitrat (pH 7), 0,1 M 2-Mercaptoethanol, 0,5 % Sarkosyl) aufgeschlossen. Die RNA wurde durch ein 5,7 M CsCl-Kissen über Nacht bei 100.000 g sedimentiert. Die polyA+-RNA enthaltende Fraktion wurde durch zweimalige Affinitätschromatographie an oligo(dT)-Cellulose abgetrennt.

Mit Hilfe des Enzyms AMV-Reverse Transkriptase und oligo(dT)12-18 als Starter wurde die polyA+-RNA in einsträngige cDNA umgeschrieben. Die Synthese des zweiten Strangs erfolgte mit E.coli-DNA-Polymerase I. An die doppelsträngige cDNA wurden mit Hilfe des Enzyms Terminale Transferase an jedem 5'-Ende ca. 10-20 dG-Reste ansynthetisiert. Ebenso wurde mit dem kommerziell erhältlichen Plasmid puC 18 verfahren, das mit der Restriktionsendonuklease SphI linearisiert wurde und an dessen 5'-Enden wie oben beschrieben ca. 10 bis 20 dC-Reste ansynthetisiert wurden. Beide DNAs wurden miteinander verschmolzen und das Hybrid in kompetente Zellen des E.coli-Stammes HB 101 transformiert. Die Klone wurden auf LB-Platten mit 100 µg/ml Ampicillin als Selektionsmarker ausplattiert und die Kolonien auf Nitrocellulose übertragen.

Die Bakterien auf den Filtern wurden repliziert, lysiert und die denaturierte DNA fest an das Filter gebunden.

Mit Hilfe eines DNA-Synthesizers (Applied Biosystems, Typ 380A) wurden vier 17er Oligonukleotidsonden mit Homologie zur publizierten DNA-Sequenz der humanen Prourokinase hergestellt. Diese Sonden hatten folgende Sequenzen:

5′ TCAGCAGCACATAGCAT 3′
5′ AACCAGGGCTGGTTCTC 3′
5′ ACCATGCACTCTTGGAC 3′
5′ TGCTGGTCACAGGTCAT 3′

Die Oligonukleotidsonden wurden am 5′-Ende mit $\gamma$-$^{32}$P-ATP markiert und die Nitrocellulosefilter in 6xSET Puffer (1xSET = 0,15 M NaCl, 0,015 M Tris-HCl pH 7,4, 0,001 M EDTA) 0,1 % SDS und 10 % Dextransulfat über Nacht bei 42°C unter Schütteln mit den Sonden inkubiert. Nach intensivem Waschen der Filter in 6 x SET/0,1 % SDS bei 42°C wurden die Filter mit einem Röntgenfilm exponiert und Klone mit Sequenzhomologie ermittelt. Die homologen Klone wurden isoliert und vereinzelt.

Einer der so isolierten Klone war pU20. Abb. 2 zeigt die Sequenz der kodierenden Region und benachbarter Bereiche.

2. Herstellung eines Hybridplasmids für die Expression der Vergleichssubstanz Prourokinase

Ausgangspunkt war das Plasmid pU20 (Beispiel 1). Aus diesem wurde durch eine limitierte TaqI/BamHI-Verdauung das Fragment des Prourokinasegens von Nukleotid 162 bis 1329 herausgeschnitten und gelelektrophoretisch isoliert (Fragment 1). Das Plasmid puc 18 wurde mit EcoRI und SalI linarisiert, dadurch entstand das Fragment 2.

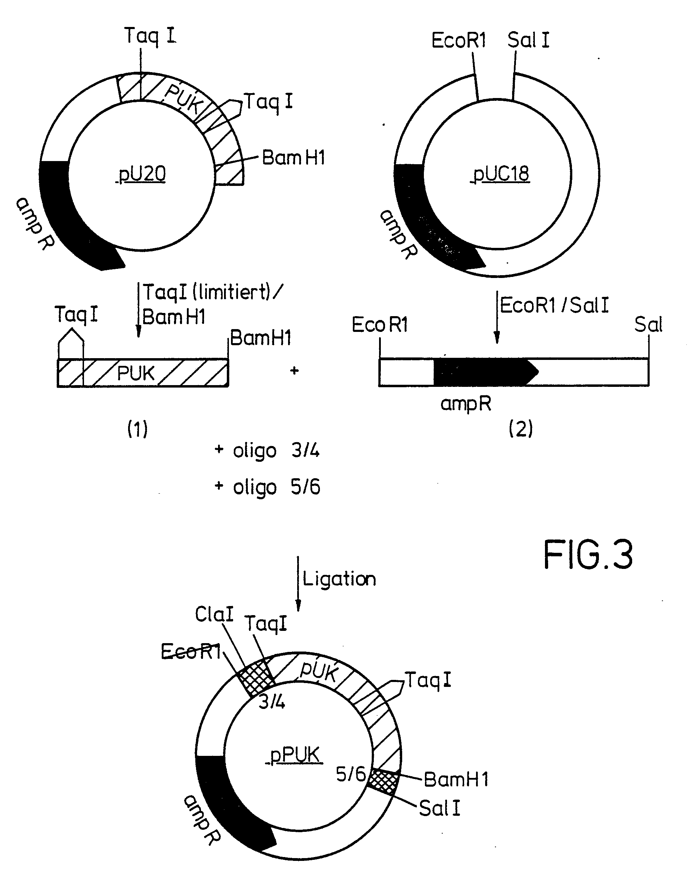

Fragmente 1 und 2 wurden mit den Oligonukleotidadaptoren 3/4 und 5/6 ligiert, die das jeweilige 5′-Ende (3/4) bzw. 3′-Ende (5/6) des Prourokinasegens wieder herstellen. Durch die Verwendung von Oligonukleotid 3/4 wurde die EcoRI-Schnittstelle am 5′-Ende deletiert. So erhielt man gemäß Abb. 3 das Hybridplasmid pPUK, das das Prourokinase-Gen als leicht klonierbare ClaI/SalI-"Kassette" zum Umsetzen in unterschiedliche Expressionsvektoren verfügbar machte.

```
            EcoRI   ClaI                                              TaqI
              |      |    Met Ser Asn Glu Leu His Gln Val Pro          |
     3/4    |AATTGAT CGAT ATG AGC AAT GAA CTT CAT CAA GTT CCA T|         3'
            |CTAGC TA TAC TCG TTA CTT GAA GTA GTT CAA GGT AGC|          5'
```

```
           BamHI
             |    Ile Arg Ser His Thr Lys Glu Glu Asn Gly Leu
    5/6  5' |G ATC CGC AGT CAC ACC AAG GAA GAG AAT GGC CTG
         3'  |GCG TCA GTG TGG TTC CTT CTC TTA CCG GAC
```

```
                  SalI
        Ala Leu Stop |
        GCC CTC TGA G|          3'
        CGG GAG ACT CAGCT|      5'
```

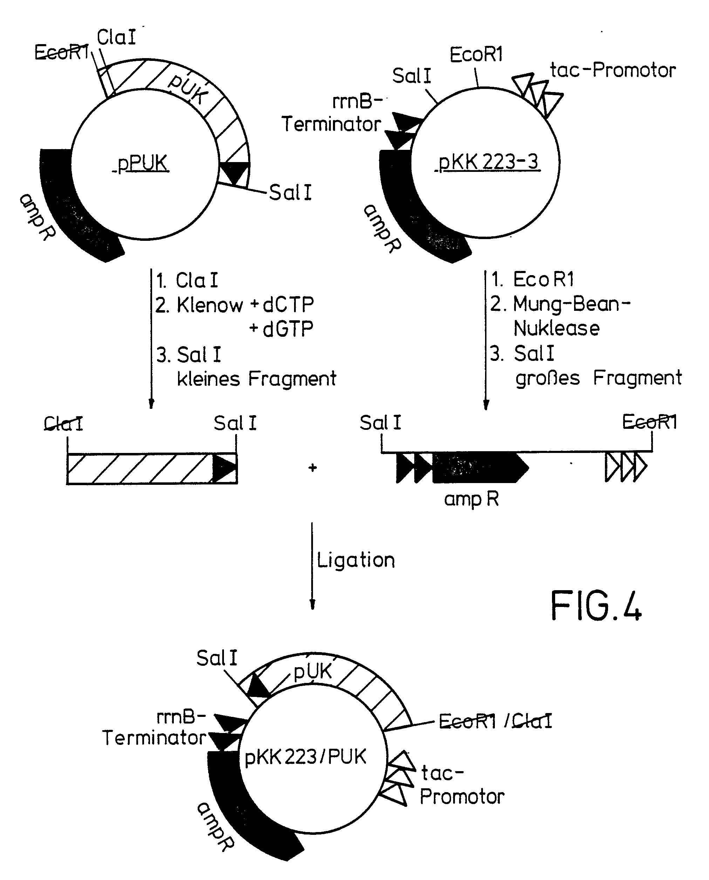

Das Hybridplasmid pPUK wurde mit ClaI geöffnet und die überstehenden 5′-Enden mit Klenow Fragment der DNA Polymerase I und dCTP und dGTP aufgefüllt. Das Fragment mit dem Urokinasegen erhielt man durch Nachschneiden mit der Endonuklease SalI und gelelektrophoretischer Aufreinigung. Dieses Fragment wurde in den kommerziell erhältlichen Expressionsvektor pKK 223-3 (Pharmacia Bestell-Nr. 27-4935-01) eingesetzt, den man mit EcoRI geöffnet, die überhängenden Enden mit "mung bean-nuclease (mung bean = Mungbohne) nach Angaben des Herstellers entfernt und anschließend mit SalI nachgeschnitten hatte. Nach der Ligation der Fragmente erhielt man das Expressionsplasmid pKK 223/PUK gemäß Abb. 4. Das Plasmid wurde in kompetent gemachte Zellen des E.coli Stammes K12 JM105 (Pharmacia Bestell-Nr. 27-1550-01) transformiert. Dieser Stamm enthält das Episom F'i$^Q$ mit dem lac-Repressor, der unter den üblichen Wachstumsbedingungen das Prourokinasegen reprimiert. Durch Einstellen des Gehalts an Isopropyl β-D-Thiogalactosid (IPTG) auf 0,5 mM in der logarithmischen Wachstumsphase wurden die Expression des Prourokinasegens induziert. Dabei fiel die Prourokinase intrazellulär denaturiert als sogenannte "Einschlußkörper" an. Diese konnten nach Aufschluß der Bakterien gereinigt und daraus die Prourokinase proteinchemisch renatuiert und aufgereinigt werden.

3. Herstellung der Hybridplasmide für die Expression von PUK mit Aminosäureaustausch in Position 156

Das Plasmid pPUK (Abb. 3, Beispiel 2) wurde mit BalI vollständig und dann mit EcoRI partiell gespalten. Das den Vektor enthaltende Fragment wurde gelelektrophoretisch aufgereinigt und mit den synthetischen Oligonukleotiden 84/85, 86/87 oder 88/89 ligiert:

```
Arg156 ──────→ Lys156

                     BalI                                    156
                       │ Gln Lys Thr Leu Arg Pro Lys Phe Lys Ile Ile
          84/85  5'    │ C CAA AAG ACT CTG CGT CCC AAG TTT AAG ATT ATT
                 3'    │ G GTT TTC TGA GAC GCA GGG GTC AAA TTC TAA TAA


                                                          EcoRI
                                          Pro Pro           │
                                          GGC GGT G└─────────┐
                                          CCG CCA CTTAA│
```

```
Arg156 ──────→ Gln156

                     BalI                                    156
                       │ Gln Lys Thr Leu Arg Pro Lys Phe Lys Ile Ile
          86/87  5'    │ C CAA AAG ACT CTG CGT CCC CAG TTT AAG ATT ATT
                 3'    │ G GTT TTC TGA GAC GCA GGG GTC AAA TTC TAA TAA


                                                          EcoRI
                                          Pro Pro           │
                                          GGC GGT G└─────────┐
                                          CCG CCA CTTAA│
```

```
Arg156 ──────→ Leu156

                     BalI                                    156
                       │ Gln Lys Thr Leu Arg Pro Lys Phe Lys Ile Ile
          88/89  5'    │ C CAA AAG ACT CTG CGT CCC CTC TTT AAG ATT ATT
                 3'    │ G GTT TTC TGA GAC GCA GGG GAG AAA TTC TAA TAA


                                                          EcoRI
                                          Pro Pro           │
                                          GGC GGT G└─────────┐
                                          CCG CCA CTTAA│
```

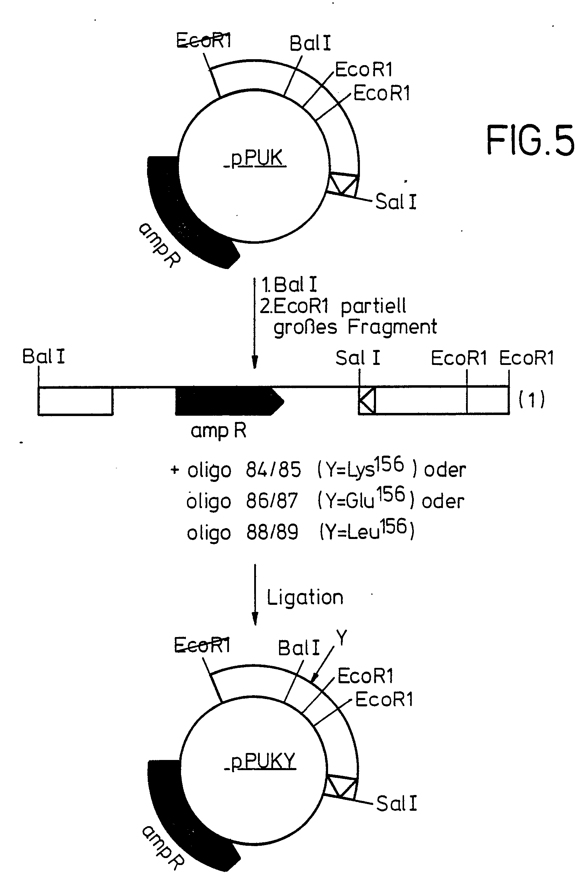

So entstanden die Hybridplasmide pPUK Lys, pPUK Gln und pPUK Leu (Abb. 5). Mit Hilfe dieser DNA-Sequenzen wurden wie in Beispiel 2 beschrieben die erfindungsgemäß beanspruchten Polypeptide gentechnologisch in E.coli hergestellt.

4. Aufreinigung und Renaturierung der in E.coli exprimierten Polypeptide

Die oben beschriebenen Polypeptide lassen sich nach ihrer Expression in E.coli nach einem Standardverfahren aufreinigen und renaturieren:

Das Zellpellet (100 g Naßgemisch) wurde in 500 ml (50 mM Phosphatpuffer pH 8,0, 10 mM EDTA, 1 mM DTT und 0,005 % Tween 80) aufgenommen und resuspendiert. Die mittels Ultraschall aufgeschlossenen Zellen wurden abzentrifugiert und das Pellet mit den Einschlußkörpern mit 50 mM Phosphatpuffer, pH 8,0, 1 M NaCl, 1 mM DTT, 0,005 % Tween 80 gewaschen.

Die gewaschenen Einschlußkörper wurden abzentrifugiert (10 min, 10.000 g) und in 6 M Guanidiniumhydrochlorid gelöst. Dies geschah durch Rühren für 30 min bei Raumtemperatur in 200 ml 50 mM Phosphatpuffer pH 8,0, 5 mM DTT, 1 mM EDTA, 6 M Guanidiniumhydrochlorid und 0,005 % Tween 80. Nicht gelöstes Protein wurde abzentrifugiert. Die Renaturierung des PUK-Muteins geschah durch Ausverdünnung dieser Lösung auf eine Proteinkonzentration zwischen 0,2 und 0,3 mg/ml in 50 mM Phosphatpuffer pH 9,0, 1,5 M Guanidiniumhydrochlorid, 0,5 M Arginin, 1 mM GSH, 0,1 mM GSSG, 1 mM EDTA, 0,005 % Tween 80.

Die Proteinreinigung erfolgte durch eine Ammoniumsulfatpräzipitation, Affinitätschromatographie und Ionenaustauschchromatographie. Dazu wurde die Renaturierungslösung mit Ammoniumsulfat (zwischen 40 bis 70 %) versetzt, das Pellet in 50 mM Phosphat, 1 M Guanidiniumhydrochlorid, 0,1 M NaCl, 0,005 % Tween 80 aufgenommen und auf eine Zn-Chelat-(Pharmacia)-Chromatographiesäule aufgetragen und mit 50 mM EDTA in obigem Puffer bei 4°C eluiert. Das eluierte Protein wurde bei 4°C gegen 10 mM Phosphat, 10 mM NaCl, 0,005 % Tween 80 dialysiert und auf eine S-Sepharose (Pharmacia) aufgetragen. Das PUK-Mutein wurde mit 50 mM Phosphat, 400 mM NaCl, 0,005 % Tween 80 von der Säule eluiert.

Die spezifischen Aktivitäten der gereinigten Proteine im direkten amidolytischen Test mit dem Substrat S-2444 (Kali-Vitrum) liegen im Bereich von 0,5 bis $1,5 \times 10^5$ U/mg.

5. Verstärkte Proteaseresistenz der PUK-Polypeptide mit Aminosäureaustausch in Pos. 156

Durch eine Thrombinspaltung der Prourokinase hinter Arg 156 entsteht ein 2 Ketten-Molekül, das nicht mehr aktivierbar ist und für eine Gerinnsel-Lyse nicht mehr zur Verfügung steht. Die oben beschriebenen Polypeptide mit Aminosäureaustausch in Pos. 156 sind nicht mehr oder nur in signifikant geringerem Ausmaß durch Thrombin inaktivierbar.

Stellvertretend für die oben beschriebenen Polypeptide soll diese verbesserte Eigenschaft für jene Moleküle demonstriert werden, die in Pos. 156 ein Glutamin oder ein Leucin tragen:

Der Thrombininaktivierungstest wurde wie folgt durchgeführt: Prourokinase (bzw. deren Muteine) wird auf 6000 U/ml verdünnt (50 mM Phosphat, pH 6,7, 300 mM NaCl, 0,005 % Tween 80) und mit Thrombin (5 U/ml) bei 37°C inkubiert. In Zeitabständen von 15 min werden Aliquote entnommen und im amidolytischen Test gemessen.

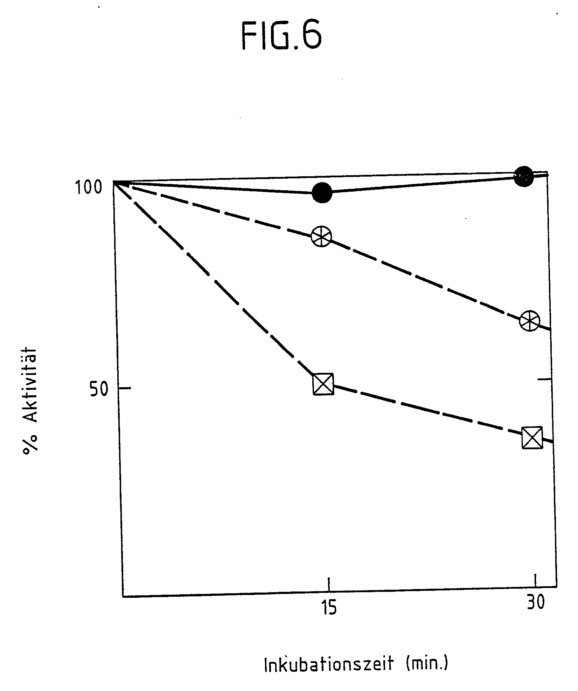

Es zeigte sich (vgl. Abb. 6), daß die Aktivität der Prourokinase im amidolytischen Test mit steigenden Thrombineinheiten abnahm. So hatte die aktivierte Prourokinase mit 2 U (x--x) Thrombin nach Inkubation von 15 min noch 86 %, nach 30 min noch 59 % seiner Ausgangsaktivität, während mit 10 U Thrombin bei gleicher Inkubationsdauer (x...x) noch 50 % bzw. 36 % der Anfangsaktivität gemessen werden konnte. Eine Inkubation der Prourokinase unter identischen Bedingungen ohne Thrombin zeigte über einen ZUeitraum von 1 h immer noch 100 % der Ausgangsaktivität. An Stelle der Prourokinase wurde nun das PUK-Mutein (Gln 156 oder Leu 156) im Thrombininhibierungstest eingesetzt. Abb. 7 macht deutlich, daß dieses Mutein nach 30 min Inkubation mit 5 U Thrombin noch 100 % der Ausgangsaktivität zeigte (o-o). Der Aminosäureaustausch an der Stelle 156 der Prourokinase (Arg→Gln) oder Leu bewirkt demnach, daß Thrombin am Mutein nicht mehr proteolytisch wirksam ist und kein inaktives Zweikettenmaterial entsteht.

**Ansprüche**

1. Polypeptide der Formel I

$R\text{-}PUK^{1-155}\text{-}X\text{-}PUK^{157-411}$

worin R ein Wasserstoffatom, Ala oder Met
$PUK^{1-155}$ und $PUK^{157-411}$ die Aminosäuren 1 bis 155 und 157 bis 411 der Prourokinase und
X Ala, Asn, Asp, Cys, Gln, Glu, Gly, His, Ile, Leu, Lys, Met, Phe, Pro, Ser, Thr, Trp, Try oder Val darstellen.

2. DNA-Sequenzen, die für die Polypeptide gemäß Anspruch 1 kodieren.

3. Vektoren, die Gensequenzen enthalten, die für die Polypeptide gemäß Anspruch 1 kodieren.

4. Gentechnologisches Verfahren zur Herstellung von Peptidsequenzen nach Anspruch 1, dadurch gekennzeichnet, daß man in einem Wirtsorganismus ein Gen zur Expression bringt, das für die Peptidsequenzen nach Anspruch 1 kodiert.

5. Arzneimittel enthaltend mindestens ein Polypeptid gemäß Anspruch 1, gegebenenfalls in einem pharmazeutisch verträglichen Träger oder Bindemittel.

6. Arzneimittel nach Anspruch 5, enthaltend die Kombination aus mindestens einem Polypeptid gemäß Anspruch 1 und einem anderen Fibrinolytikum.

Abb. 1: Aminosäuresequenz der reifen Prourokinase

```
                                       10
   Ser-Asn-Glu-Leu-His-Gln-Val-Pro-Ser-Asn-
                                       20
   Cys-Asp-Cys-Leu-Asn-Gly-Gly-Thr-Cys-Val-
                                       30
   Ser-Asn-Lys-Tyr-Phe-Ser-Asn-Ile-His-Trp-
                                       40
   Cys-Asn-Cys-Pro-Lys-Lys-Phe-Gly-Gly-Gln-
                                       50
   His-Cys-Glu-Ile-Asp-Lys-Ser-Lys-Thr-Cys-
                                       60
   Tyr-Glu-Gly-Asn-Gly-His-Phe-Tyr-Arg-Gly-
                                       70
   Lys-Ala-Ser-Thr-Asp-Thr-Met-Gly-Arg-Pro-
                                       80
   Cys-Leu-Pro-Trp-Asn-Ser-Ala-Thr-Val-Leu-
                                       90
   Gln-Gln-Thr-Tyr-His-Ala-His-Arg-Ser-Asp-
                                       100
   Ala-Leu-Gln-Leu-Gly-Leu-Gly-Lys-His-Asn-
                                       110
   Tyr-Cys-Arg-Asn-Pro-Asp-Asn-Arg-Arg-Arg-
                                       120
   Pro-Trp-Cys-Tyr-Val-Gln-Val-Gly-Leu-Lys-
                                       130
   Pro-Leu-Val-Gln-Glu-Cys-Met-Val-His-Asp-
                                       140
   Cys-Ala-Asp-Gly-Lys-Lys-Pro-Ser-Ser-Pro-
                                       150
   Pro-Glu-Glu-Leu-Lys-Phe-Gln-Cys-Gly-Gln-
                                       160
   Lys-Thr-Leu-Arg-Pro-Arg-Phe-Lys-Ile-Ile-
                                       170
   Gly-Gly-Glu-Phe-Thr-Thr-Ile-Glu-Asn-Gln-
                                       180
   Pro-Trp-Phe-Ala-Ala-Ile-Tyr-Arg-Arg-His-
                                       190
   Arg-Gly-Gly-Ser-Val-Thr-Tyr-Val-Cys-Gly-
                                       200
   Gly-Ser-Leu-Ile-Ser-Pro-Cys-Trp-Val-Ile-
                                       210
   Ser-Ala-Thr-His-Cys-Phe-Ile-Asp-Tyr-Pro-
```

EP 0 312 942 A2

BASF Aktiengesellschaft    12    870557    O.Z. 0050/39531

Abb. 1 (Fortsetzung)

```
                                      220
    Lys-Lys-Glu-Asp-Tyr-Ile-Val-Tyr-Leu-Gly-
                                      230
    Arg-Ser-Arg-Leu-Asn-Ser-Asn-Thr-Gln-Gly-
                                      240
    Glu-Met-Lys-Phe-Glu-Val-Glu-Asn-Leu-Ile-
                                      250
    Leu-His-Lys-Asp-Tyr-Ser-Ala-Asp-Thr-Leu-
                                      260
    Ala-His-His-Asn-Asp-Ile-Ala-Leu-Leu-Lys-
                                      270
    Ile-Arg-Ser-Lys-Glu-Gly-Arg-Cys-Ala-Gln-
                                      280
    Pro-Ser-Arg-Thr-Ile-Gln-Thr-Ile-Cys-Leu-
                                      290
    Pro-Ser-Met-Tyr-Asn-Asp-Pro-Gln-Phe-Gly-
                                      300
    Thr-Ser-Cys-Glu-Ile-Thr-Gly-Phe-Gly-Lys-
                                      310
    Glu-Asn-Ser-Thr-Asp-Tyr-Leu-Tyr-Pro-Glu-
                                      320
    Gln-Leu-Lys-Met-Thr-Val-Val-Lys-Leu-Ile-
                                      330
    Ser-His-Arg-Glu-Cys-Gln-Gln-Pro-His-Tyr-
                                      340
    Tyr-Gly-Ser-Glu-Val-Thr-Thr-Lys-Met-Leu-
                                      350
    Cys-Ala-Ala-Asp-Pro-Gln-Trp-Lys-Thr-Asp-
                                      360
    Ser-Cys-Gln-Gly-Asp-Ser-Gly-Gly-Pro-Leu-
                                      370
    Val-Cys-Ser-Leu-Gln-Gly-Arg-Met-Thr-Leu-
                                      380
    Thr-Gly-Ile-Val-Ser-Trp-Gly-Arg-Gly-Cys-
                                      390
    Ala-Leu-Lys-Asp-Lys-Pro-Gly-Val-Tyr-Thr-
                                      400
    Arg-Val-Ser-His-Phe-Leu-Pro-Trp-Ile-Arg-
                                      410
    Ser-His-Thr-Lys-Glu-Glu-Asn-Gly-Leu-Ala-
    411
    Leu
```

Abb. 2 EP 0 312 942 A2 ).Z. 3C5C/39531

```
      gtccccgcagcgccgtcgcgccctcctgccgcaggccaccgaggccgccgccgtctagcg
  1   ---------+---------+---------+---------+---------+---------+  60
      cagggcgtcgcggcagcgcgggaggacggcgtccggtggctccggcggcggcagatcgc


      ccccgacctcgccaccatgagagccctgctggcgcgcctgcttctctgcgtcctggtcgt
 61   ---------+---------+---------+---------+---------+---------+  120
      ggggctggagcggtggtactctcgggacgaccgcgcggacgaagagacgcaggaccagca


                                   T
                                   a
                                   q
                                   I
      gagcgactccaaaggcagcaatgaacttcatcaagttccatcgaactgtgactgtctaaa
121   ---------+---------+---------+---------+---------+---------+  180
      ctcgctgaggtttccgtcgttacttgaagtagttcaaggtagcttgacactgacagatt
         SerAsnGluLeuHisGlnValProSerAsnCysAspCysLeuAsn -


      tggaggaacatgtgtgtccaacaagtacttctccaacattcactggtgcaactgcccaaa
181   ---------+---------+---------+---------+---------+---------+  240
      acctccttgtacacacaggttgttcatgaagaggttgtaagtgaccacgttgacgggttt
      GlyGlyThrCysValSerAsnLysTyrPheSerAsnIleHisTrpCysAsnCysProLys -


      gaaattcggagggcagcactgtgaaatagataagtcaaaaacctgctatgaggggaatgg
241   ---------+---------+---------+---------+---------+---------+  300
      ctttaagcctcccgtcgtgacactttatctattcagtttttggacgatactcccttacc
      LysPheGlyGlyGlnHisCysGluIleAspLysSerLysThrCysTyrGluGlyAsnGly -


      tcacttttaccgaggaaaggccagcactgacaccatgggccggccctgcctgccctggaa
301   ---------+---------+---------+---------+---------+---------+  360
      agtgaaaatggctcctttccggtcgtgactgtggtacccggccgggacggacggacctt
      HisPheTyrArgGlyLysAlaSerThrAspThrMetGlyArgProCysLeuProTrpAsn -


      ctctgccaatgtccttcagcaaacgtaccatgcccacagatctgatgctcttcagctggg
361   ---------+---------+---------+---------+---------+---------+  420
      gagacggttacaggaagtcgtttgcatggtacgggtgtctagactacgagaagtcgaccc
      SerAlaAsnValLeuGlnGlnThrTyrHisAlaHisArgSerAspAlaLeuGlnLeuGly -


      cctggggaaacataattactgcaggaacccagacaaccggaggcgaccctggtgctatgt
421   ---------+---------+---------+---------+---------+---------+  480
      ggacccctttgtattaatgacgtccttgggtctgttggcctccgctgggaccacgataca
      LeuGlyLysHisAsnTyrCysArgAsnProAspAsnArgArgArgProTrpCysTyrVal -
```

Abb. 2 Forts.

EP 0 312 942 A2                         O.Z. 0050/39531

```
      gcaggtgggcctaaagccgcttgtccaagagtgcatggtgcatgactgcgcagatggaaa
481   ---------+---------+---------+---------+---------+---------+ 540
      cgtccacccggatttcggcgaacaggttctcacgtaccacgtactgacgcgtctaccttt

      GlnValGlyLeuLysProLeuValGlnGluCysMetValHisAspCysAlaAspGlyLys -


                                        B
                                        a
                                        l
                                        I
      aaagccctcctctcctccagaagaattaaaatttcagtgtggccaaaagactctgaggcc
541   ---------+---------+---------+---------+---------+---------+ 600
      tttcgggaggagaggaggtcttcttaattttaaagtcacaccggttttctgagactccgg

      LysProSerSerProProGluGluLeuLysPheGlnCysGlyGlnLysThrLeuArgPro -


                   E                    T
                   c                    a
                   o                    q
                   R                    I
                   I
      ccgctttaagattattggggggagaattcaccaccatcgagaaccagccctggtttgcggc
601   ---------+---------+---------+---------+---------+---------+ 660
      ggcgaaattctaataacccccctcttaagtggtggtagctcttggtcgggaccaaacgccg

      ArgPheLysIleIleGlyGlyGluPheThrThrIleGluAsnGlnProTrpPheAlaAla -

      catctacaggaggcaccgggggggctctgtcacctacgtgtgtggaggcagcctcatcag
661   ---------+---------+---------+---------+---------+---------+ 720
      gtagatgtcctccgtggcccccccgagacagtggatgcacacacctccgtcggagtagtc

      IleTyrArgArgHisArgGlyGlySerValThrTyrValCysGlyGlySerLeuIleSer -

      cccttgctgggtgatcagcgccacacactgcttcattgattacccaaagaaggaggacta
721   ---------+---------+---------+---------+---------+---------+ 780
      gggaacgacccactagtcgcggtgtgtgacgaagtaactaatgggtttcttcctcctgat

      ProCysTrpValIleSerAlaThrHisCysPheIleAspTyrProLysLysGluAspTyr -

      catcgtctacctgggtcgctcaaggcttaactccaacacgcaaggggagatgaagtttga
781   ---------+---------+---------+---------+---------+---------+ 840
      gtagcagatggacccagcgagttccgaattgaggttgtgcgttcccctctacttcaaact

      IleValTyrLeuGlyArgSerArgLeuAsnSerAsnThrGlnGlyGluMetLysPheGlu -

      ggtggaaaacctcatcctacacaaggactacagcgctgacacgcttgctcaccacaacga
841   ---------+---------+---------+---------+---------+---------+ 900
      ccaccttttggagtaggatgtgttcctgatgtcgcgactgtgcgaacgagtggtgttgct

      ValGluAsnLeuIleLeuHisLysAspTyrSerAlaAspThrLeuAlaHisHisAsnAsp -

      cattgccttgctgaagatccgttccaaggagggcaggtgtgcgcagccatcccggactat
901   ---------+---------+---------+---------+---------+---------+ 960
      gtaacggaacgacttctaggcaaggttcctcccgtccacacgcgtcggtagggcctgata

      IleAlaLeuLeuLysIleArgSerLysGluGlyArgCysAlaGlnProSerArgThrIle -
```

Abb. 2 Forts.

EP 0 312 942 A2

C.Z. 0050/39531

```
                                    T
                                    a
                                    q
                                    I
        acagaccatctgcctgccctcgatgtataacgatccccagtttggcacaagctgtgagat
    961 ---------+---------+---------+---------+---------+---------+ 1020
        tgtctggtagacggacgggagctacatattgctaggggtcaaaccgtgttcgacactcta

        GlnThrIleCysLeuProSerMetTyrAsnAspProGlnPheGlyThrSerCysGluIle -

                                    E
                                    c
                                    o
                                    R
                                    I
        cactggctttggaaaagagaattctaccgactatctctatccggagcagctgaaaatgac
   1021 ---------+---------+---------+---------+---------+---------+ 1080
        gtgaccgaaacctttttctcttaagatggctgatagagataggcctcgtcgacttttactg

        ThrGlyPheGlyLysGluAsnSerThrAspTyrLeuTyrProGluGlnLeuLysMetThr -

        tgttgtgaagctgatttcccaccgggagtgtcagcagccccactactacggctctgaagt
   1081 ---------+---------+---------+---------+---------+---------+ 1140
        acaacacttcgactaaagggtggccctcacagtcgtcggggtgatgatgccgagacttca

        ValValLysLeuIleSerHisArgGluCysGlnGlnProHisTyrTyrGlySerGluVal -

        caccaccaaaatgctgtgtgctgctgacccacagtggaaaacagattcctgccagggaga
   1141 ---------+---------+---------+---------+---------+---------+ 1200
        gtggtggttttacgacacacgacgactgggtgtcaccttttgtctaaggacggtccctct

        ThrThrLysMetLeuCysAlaAlaAspProGlnTrpLysThrAspSerCysGlnGlyAsp -

        ctcaggggggacccctcgtctgttccctccaaggccgcatgactttgactggaattgtgag
   1201 ---------+---------+---------+---------+---------+---------+ 1260
        gagtccccctggggagcagacaagggaggttccggcgtactgaaactgaccttaacactc

        SerGlyGlyProLeuValCysSerLeuGlnGlyArgMetThrLeuThrGlyIleValSer -

        ctggggccgtggatgtgccctgaaggacaagccaggcgtctacacgagagtctcacactt
   1261 ---------+---------+---------+---------+---------+---------+ 1320
        gaccccggcacctacacgggacttcctgttcggtccgcagatgtgctctcagagtgtgaa

        TrpGlyArgGlyCysAlaLeuLysAspLysProGlyValTyrThrArgValSerHisPhe -

                                    B
                                    a
                                    m
                                    H
                                    I
        cttaccctggatccgcagtcacaccaaggaagagaatggcctggccctctgagggtcccc
   1321 ---------+---------+---------+---------+---------+---------+ 1380
        gaatgggacctaggcgtcagtgtggttccttctcttaccggaccgggagactcccagggg

        LeuProTrpIleArgSerHisThrLysGluGluAsnGlyLeuAlaLeuEnd
```

Abb. 2 Forts. EP 0 312 942 A2 C.I. CC5C/3953:

```
          agggaggaaacgggcaccacccgctttcttgctggttgtcatttttgcagtagagtcatc
     1381  --------+---------+---------+---------+---------+---------+  1440
          tccctcctttgcccgtggtgggcgaaagaacgaccaacagtaaaaacgtcatctcagtag


          tccatcagctgtaagaagagactgggaagataggctctgcacagatggatttgcctgtgc
     1441  --------+---------+---------+---------+---------+---------+  1500
          aggtagtcgacattcttctctgacccttctatccgagacgtgtctacctaaacggacacg
```

O.Z. 0050/39531

FIG.3

0050/39531

FIG.4

FIG.5

# FIG.6

%  Aktivität

100

50

15    30

Inkubationszeit (min.)